# EUROPÄISCHE PATENTSCHRIFT

(11) **EP 2 056 781 B1**
(45) Veröffentlichungstag und Bekanntmachung des Hinweises auf die Patenterteilung: **03.11.2010**
(21) Anmeldenummer: 07785102.0
(22) Anmeldetag: 20.08.2007
(51) Int. Cl.: A61K 8/31, A61K 8/34, A61K 8/42, A61K 8/81, A61Q 19/00

(54) **WASSERFREIE, HARNSTOFFHALTIGE, DERMATOLOGISCHE ODER KOSMETISCHE ZUBEREITUNG**
ANHYDROUS DERMATOLOGICAL OR COSMETIC PREPARATION COMPRISING UREA
PRÉPARATION DERMATOLOGIQUE OU COSMÉTIQUE EXEMPTE D'EAU, CONTENANT DE L'URÉE

(30) Priorität: 28.08.2006 CH 13712006
(43) Veröffentlichungstag der Anmeldung: 13.05.2009
(73) Patentinhaber: Suess, Hans, R., CH-4656 Starrkirch-Wil (CH)
(72) Erfinder: Suess, Hans, R., CH-4656 Starrkirch-Wil (CH)
(74) Vertreter: Frei Patent Attorneys
(86) Internationale Anmeldenummer: PCT/CH2007/000407
(87) Internationale Veröffentlichungsnummer: WO 2008/025176

(56) Entgegenhaltungen:
- WO-A-02/47643
- GB-A- 2 157 173
- US-A1- 2003 228 335

## Beschreibung

Die Erfindung betrifft eine dermatologische oder kosmetische Zubereitung nach dem Oberbegriff des ersten unabhängigen Patentanspruchs. Die Zubereitung ist wasserfrei und enthält Harnstoff.

Harnstoff (Urea, Carbamid, CAS No. 57-13-6) ist im Körper von Säugern als Endprodukt des Eiweissabbaus enthalten und ist deshalb für die meisten pathogenen Mikroben kein Energielieferant. Harnstoff ist im Urin enthalten und hat eine antimikrobielle Wirkung. Wohl aus diesem Grunde wurde Urin im alten Babylon in entzündete Wunden gegeben. Harnstoff ist ferner enthalten in der Hornschicht der Haut als Bestandteil des Feuchthaltefaktors (NMF), der für einen ausreichenden Wassergehalt der Hornschicht verantwortlich ist und von dem der Harnstoff etwa 7% ausmacht. Wenn die Hornschicht einen zu geringen Gehalt an Harnstoff aufweist, ist das Wasserbindungsvermögen reduziert und der transepidermale Wasserverlust steigt an. Dadurch wird die Haut trocken, schuppig und irritationsanfällig. Bei verschiedenen Hautkrankheiten wie Psoriasis weist die Hornschicht einen stark verminderten Wassergehalt auf. Dieser ist beispielsweise bei Neurodermitis (Atopische Dermatitis) bis zu über 80% vermindert. Es macht deshalb Sinn, trockene Haut direkt mit Harnstoff zu versorgen.

Harnstoff ist bipolar und deshalb gut wasserlöslich und zu Interaktionen mit ionischen Salzlösungen befähigt. Da Harnstoff in Lipiden nicht löslich ist, wird er meist in Emulsionen eingesetzt, wobei O/W-Emulsionen (Oel in Wasser) schneller in die Haut eindringen als W/O-Emulsionen (Wasser in Oel). In wasserhaltigen Zubereitungen zersetzt sich aber der Harnstoff langsam zu Kohlendioxid und Ammoniak. Dieser Abbau kann durch Lagerung bei tiefen Temperaturen und/oder durch Zusatz von Säuren, z.B. Hydroxysäuren wie Glykolsäure oder Milchsäure verlangsamt aber nicht völlig verhindert werden. Ebenfalls bekannte, harnstoffhaltigen Zubereitungen, die wasserfrei sind, weisen den Nachteil des Harnstoffabbaus nicht auf. Solche Zubereitungen werden zwar langsamer von der Haut aufgenommen als Emulsionen, erhöhen jedoch den Feuchtigkeitsgehalt der Hornschicht stärker. Dies ist darauf zurückzuführen, dass die wasserfreien Zubereitungen zusätzlich zur hydratisierenden Wirkung des Harnstoffs auch eine okkludierende Wirkung haben.

Eine wasserfreie, harnstoffhaltige Zubereitung (emplastrum urea) wurde schon 1960 in Russland von Arievich zur Ablösung von durch Pilzbefall zerstörten Nägeln verwendet (Cutis 25:605-612, 1980). Die wasserfreie, harnstoffhaltige Zubereitung gemäss Arievich enthält 40% Harnstoff, 25% weisse Vaseline, 20% Lanolin USP, 5% Bienenwachs und 10% Silicagel. Um eine Körnigkeit des Präparates zu vermeiden ist der Harnstoff fein gemahlen.

In der Patentanmeldung GP 2 157 173 (Rhoem Pharma GmbH, Deutschland; 1984) wird ein wasserfreier Stift beschrieben, der aus Harnstoff und vorwiegend inerten Lipiden wie Paraffin besteht.

In der Patentanmeldung WO 02/47643 wird eine wasserfreie, harnstoffhaltige Zubereitung beschrieben, die als Salbengrundlage Vaseline, Paraffinöl und/oder mikrokristalline Wachse enthält. In der Herstellungsbeschreibung wird darauf hingewiesen, dass es wichtig sei, möglichst wasserfrei zu arbeiten, da der in einer hydrophoben Phase dispergierte Harnstoff oftmals eine Tendenz zu Rekristallisation habe, was sich auf der Haut als deutlicher und unangenehmer "Sandpapier"-Effekt bemerkbar mache und was wohl der Grund dafür sei, dass solche wasserfreien Zubereitungen kaum auf dem Markte zu finden seien.

Es ist zu vermuten, dass der genannte Sandpapier-Effekt jedoch eher auf Inklusionskomplexen aus Harnstoff und daran angelagerten, unverzweigten aliphatischen Verbindungen beruht als auf rekästallisiertem Harnstoff. Friederich Bengen hat in der Patentschrift DE-12438 (1940) schon solche Inklusionskomplexe oder Additionsprodukte beschrieben. Lineare (unverzweigte oder geradkettige) aliphatische Kohlenwasserstoffe mit mindestens 6 und höchstens 50 C-Atomen bilden definierte Komplexe mit Harnstoff, während verzweigte oder zyklische Verbindungen dies normalerweise nicht tun. Dies geht auch hervor aus W.J. Zimmerschied et al., "Cristalline Adducts of Urea with Linear Aliphatic Compounds", Ind. Eng. Chem: 42:1300-1306 (1950).

Ferner ist es bekannt, dass die Tendenz, Hautreizungen zu verursachen, für harnstoffhaltige Präparate mit steigendem Harnstoffgehalt steigt.

Die Erfindung stellt sich nun die Aufgabe, eine wasserfreie, harnstoffhaltige, dermatologische oder kosmetische Zubereitung zu schaffen, die bei relativ niedrigem Harnstoffgehalt und deshalb ohne die oben genannten Hautreizungen das Wasserrückhaltevermögen der Hornschicht relevant zu verbessern vermag und den ebenfalls oben genannten Sandpapier-Effekt nicht aufweist Es geht also darum, insbesondere das Hautgefühl (hautsensorische Eigenschaften) von bekannten wasserfreien, harnstoffhaltigen Zubereitungen zu verbessern, ohne deren Wirkung auf das Wasserrtickhaltevermögen der Hornschicht der Haut zu schmälern. Dies ist insbesondere wichtig, da eine Zubereitung, auch wenn sie dermatologisch noch so wirksam ist, keine Verwendungstreue (Compliance) findet und dadurch ihre gute Wirkung nicht entfalten kann, wenn sie keine guten hautsensorischen Eigenschaften aufweist.

Die gestellte Aufgabe wird gelöst durch die wasserfreie, harnstoffhaltige, dermatologische oder kosmetische Zubereitung, wie sie im ersten unabhängigen Anspruch definiert ist.

Erfindungsgemäss enthält die wasserfreie, harnstoffhaltige Zubereitung als zusätzlichen Wirkstoff Phytantriol (CAS-No. 74563-64-7; 3,7,11,15-Tetramethylhexadecan-1,2,3-triol) und weist vorteilhafterweise eine gelartige Salbenbasis auf, die im wesentlichen frei ist von unverzweigten Kohlenwasserstoffen mit 6 bis 50 C-Atomen, das heisst weniger als 5 Gew.% (vorzugsweise weniger als 2 Gew.%) solcher Kohlenwasserstoffe enthält.

Durch die Zugabe von Phytantriol, das selbst ein das Wasserrückhaltevermögen der Hornschicht steigernde Wirkung hat, kann auch mit Hamstoffkonzentrationen, die tief genug sind, um nicht zu Hautreizungen zu führen, eine hohe Wirkung erzielt werden. Da Phytantriol bekannterweise die Aufnahmefähigkeit der Haut für Aminosäuren steigert, ist anzunehmen, dass es auch die Aufnahmefähigkeit von Harnstoff steigert, was ein weiterer Vorteil der erfindungsgemässen Zubereitung ausmacht. Phytantriol zeigt ferner eine hohe Lipidlöslichkeit und dank seiner Verzweigtheit keine Tendenz zur Bildung von Anlagerungskomplexen mit dem Harnstoff.

Phytantriol verbessert die hautsensorischen Eigenschaften auch dadurch, dass es die Adhäsion der Zubereitung gegenüber hautfremden Substraten herabsetzt. Dadurch wird die aufgetragene Zubereitung kaum noch abgetragen und bleibt deshalb länger auf der Haut. Dies ergibt eine weitere Wirkungssteigerung, die sowohl ökologisch als auch ökonomisch sinnvoll ist.

Durch die Wahl der genannten Salbenbasis, die vorteilhafterweise aus mindestens einem flüssigen, verzweigten, vorzugsweise aliphatischen Kohlenwasserstoff und mindestens einem festen, polymeren Kohlenwasserstoff (mit verzweigten oder unverzweigten Ketten und mit mehr als 50 C-Atomen) besteht, werden die oben genannten Hamstoftkomplexe und dadurch der Sandpapier-Effekt vermieden.

Die erfindungsgemässe Zubereitung enthält zwischen 0,1 bis 10 Gew.%, insbesondere 0,2 bis 5 Gew.%, vorzugsweise 2 Gew.% Phytantriol und zwischen 3 und 50 Gew.%, insbesondere zwischen 3 und 20 Gew.%, vorzugsweise zwischen 5 und 10 Gew.% Harnstoff.

Die erfindungsgemässe Zubereitung kann ferner weitere mikronisierte Wirkstoffe wie beispielsweise Pigmente oder UV-Filter enthalten und/oder im flüssigen verzweigtkettigen Kohlenwasserstoff gelöste kosmetische oder dermatologische Wirkstoffe.

Phytantriol ist ein Tetraisoprenoid. Isopren ist der Grundbaustein von vielen Naturprodukten wie beispielsweise von Terpenen, Steroiden oder Kautschuk. Isoprenoide Anteile sind z.B. auch in Vitaminen der A-Gruppe sowie im Chlorophyll enthalten. Phytan kommt auch in der Leber, in Oelschiefer und in anderen Sedimenten und auch in Meteoriten vor (Kates, M. Biochemistry 1967, 6, 3329). Der dem Phytan entsprechende Alkohol Phytanol ist Bestandteil eines menschlichen Enzyms. Ferner besteht die Zellhülle der Archaea aus Aethern von Phytanol mit Glycerin, die wesentlich hydrophober sind als die Triglyceride der epidermalen Barriere der Säuger.

Als flüssiger, verzweigtkettiger Kohlenwasserstoff in der Salbenbasis der erfindungsgemässen Zubereitung eigenen sich beispielsweise: Isoparaffin C11-C13 (Isopar L, Exxon), Paraffinöl (Mineral Oil, CAS Nr. 80122-95-1, 8020-83-5 oder 8042-47-5), Hydrierte Polydecene (CAS Nr. 25189-70 oder 37309-58-3), Isododecane (CAS Nr. 141-70-8, 13475-82-6 oder 31807-55-3) Isohexadecane (CAS Nr. 4390-04-9 oder 60908-77-2), Isoeicosane (CAS Nr. 52845-07-5) oder Mischungen aus mindestens zwei der genannten Kohlenwasserstoffe.

Als fester, polymerer Kohlenwasserstoff in der Salbenbasis der erfindungsgemässen Zubereitung eignen sich beispielsweise Polyethylene (CAS Nr. 9002-88-4), Polypropylene (CAS Nr. 9003-07-0), Polybutene (CAS Nr. 9003-28-5 oder 9003-29-6), Polyisobutene (CAS Nr. 9003-31-0), Polystyrole (CAS Nr. 9003-53-06), Ethylenpropylene (CAS Nr. 9010-79-1) oder entsprechende Mischpolymere oder Mischungen aus mindestens zwei der genannten Polymere.

Der flüssige, verzweigtkettige Kohlenwasserstoff wird mit dem festen, polymeren Kohlenwasserstoff verdickt, wobei ein salbenartiges Gel entsteht.

Eine beispielhafte Formulierung enthält 84 Gew.% Isopar L (Exxon), 9 Gew.% Polyethylen (z.B. Luwax A, BASF), 5 Gew.% Harnstoff (pharm. Qualität) und 2 Gew.% Phytantriol.

Zur Herstellung der erfindungsgemässen Zubereitung wird vorzugsweise in den flüssigen, verzweigten, vozugsweise aliphatischen Kohlenwasserstoff bei einer Temperatur von etwa 125°C der feste, polymere Kohlenwasserstoff eingetragen und die Mischung wird gerührt bis sich ein klares Gel bildet. Dann wird bei 110 bis 115°C, also deutlich unter dem Schmelzpunkt von Harnstoff (133°C) der mikronisierte Harnstoff und das Phytantriol eingetragen und hernach unter stetem Rühren auf Zimmertemperatur abgekühlt. Der mikronisierte Harnstoff kann bei Umgebungstemperatur und bei normaler Luftfeuchtigkeit ohne Zusatz eines Trockenhaltemittels beispielsweise in einer offenen Stiftmühle (z.B. Typ 160 von Alpine Augsburg) hergestellt werden.

Die nach dem obigen Rezept hergestellte Zubereitung bleibt bei einer Lagerung zwischen 15 und 30°C für mindestens ein Jahr vollkommen homogen, auch wenn zu deren Herstellung ein handelsübliches Phytantriol mit bis zu 0,5% Wassergehalt verwendet wird.

### Beispiele (Zusammensetzung in Gew.%)

| **Zubereitung Nr.** | **1 Vergl** | **2** | **3 Vergl** | **4 Vergl** | **5** | **6** | **7** | **8** |
|---|---|---|---|---|---|---|---|---|
| C11-C13 Isopraffin | | | | | 79 | | | |
| Paraffinöl | | | | | | 79 | | |
| Polydecen | | | | | | | | 81 |
| Isododecan | 16 | 16 | 16 | 16 | | | | |
| Isohexadecan | | | | | | | 81 | |
| Isoeicosan | 65 | 65 | 65 | 65 | | | | |
| Polyethylen | 12 | 12 | 12 | 12 | 9 | 9 | 12 | 12 |
| Harnstoff, mikronisiert | 5 | 5 | 5 | 5 | 10 | 10 | 5 | 5 |
| Phytantriol | | 2 | | | 2 | 2 | 2 | 2 |
| Glyceryloleat | | | 2 | | | | | |
| Glycerylisostearat | | | | 2 | | | | |

Für die in der obigen Tabelle aufgeführten Zubereitungsbeispiele wurden hautsensorische Versuche durchgeführt. Die Bestimmung der hautsensorischen Eigenschaften der Zubereitungen wurden nach den Wegleitungen von Morten Meilgaard, Gail Vance Civille und Thomas Carr ("Sensory Evaluation Techniques", 3rd Edition 1999, CRC Press, Seiten 184-186) durchgeführt und erbrachten die folgenden Resultate:
- Die Zubereitung 2, die sich von der Vergleichs-Zubereitung 1 nur durch den Gehalt an Phytantriol unterscheidet, bewirkt ein im Vergleich zur Zubereitung 1 relevant verbessertes Hautgefühl. Das Hautgefühl der Zubereitung 2 ist angenehmer als das Hautgefühl von entsprechenden O/W-Emulsionen.
- Die Vergleichs-Zubereitung 3, die anstelle von Phytantriol Glyceryloleat enthält, ergibt keine Verbesserung des Hautgefühls ähnlich wie die Vergleichs-Zubereitung 1. Phytantriol, das in seiner handlesüblichen Form 0,5% Wasser enthält, bildet mit Wasser spontan kubisch hochgeordnete Flüssigkristalle im Nanobereich, in welche dermatologische Wirkstoffe eingearbeitet werden können, die dadurch ein erhöhtes Penetrationsvermögen erhalten. Der Vergleich der Zubereitung 2 mit der Vergleichs-Zubereitung 3, die ebenfalls solche kubischen Systeme bildendes Glyceryloleat enthält, weist darauf hin, dass die durch das Phytantriol erwirkte Verbesserung der hautsensorischen Eigenschaften wohl nicht auf diese Systeme zurückzuführen ist.
- Die Vergleichs-Zubereitungen 4, in der gegenüber der Zubereitung 2 das Phytantriol mit Glycerylisostearat ersetzt ist, zeigt gegenüber der Vergleichs-Zubereitung 3 eine schwache Verbesserung des Hautgefühls, die wohl auf die Verzweigtheit des Isostearates zurückzuführen ist.
- Auch die Zubereitungen mit hohen Harnstoffgehalten (Zubereitungen 5 und 6: 10%) lassen sich ohne unangenehme Nebenerscheinungen wie Stechen oder Brennen auf die Gesichtshaut auftragen.
- Für Zubereitungen mit dem niederviskosen Isoparaffin (Zubereitung 5) stellt sich die Verbesserung der sensorischen Eigenschaften rascher ein als für Zubereitungen mit höher vislcosem Paraffinöl, was auf die Erhöhung der Gleitfähigkeit beim Bestreichen der Haut und auf eine Abnahme der fettigen Eigenschaften zurückzuführen ist. Dasselbe Resultat ergibt sich beim Vergleich der Zubereitung 7, die nieder viskoses Isohexadecan enthält, mit der Zubereitung 8, die viskoseres Polydecen enthält.

## Patentansprüche

1. Wasserfreie, dermatologische oder kosmetische Zubereitung, die Harnstoff enthält, **dadurch gekennzeichnet, dass** sie Phytantriol enthält.

2. Zubereitung nach Anspruch 1, **dadurch gekennzeichnet, dass** sie eine gelartige Basis aufweist, die im wesentlichen frei ist von unverzweigten Kohlenwasserstoffen mit 5 bis 50 Kohlenstoffatomen.

3. Zubereitung nach Anspruch 2, **dadurch gekennzeichnet, dass** die gelartige Basis mindestens einen flüssigen, verzweigtkettigen Kohlenwasserstoff und mindestens einen festen polymeren Kohlenwasserstoff enthält.

4. Zubereitung nach einem der Ansprüche 1 bis 3, **dadurch gekennzeichnet, dass** der Harnstoff mikronisiert ist.

5. Zubereitung nach einem der Ansprüche 1 bis 4, **dadurch gekennzeichnet, dass** das Phytantriol in der Zubereitung eine Konzentration zwischen 0,1 und 10 Gew.% hat.

6. Zubereitung nach einem der Ansprüche 1 bis 4, **dadurch gekennzeichnet, dass** das Phytantriol in der Zubereitung eine Konzentration von 0,2 bis 3 Gew.% hat.

7. Zubereitung nach einem der Ansprüche 1 bis 6, **dadurch gekennzeichnet, dass** der Harnstoff in der Zubereitung eine Konzentration von 3 bis 50 Gew.% hat.

8. Zubereitung nach einem der Ansprüche 1 bis 6, **dadurch gekennzeichnet, dass** der Harnstoff in der Zubereitung eine Konzentration von 3 bis 20 Gew.% hat.

9. Zubereitung nach einem der Ansprüche 1 bis 8, **dadurch gekennzeichnet, dass** der verzweigte aliphatische Kohlenwasserstoff Isoparaffin C11-C13, Paraffinöl, ein hydriertes Polydecen, ein Isododecan, ein Isohexadecan, ein Isoeicosan oder eine Mischung davon ist und dass der feste, polymere Kohlenwasserstoff ein Polyethylen, ein Polypropylen, ein Polybuten, ein Polyisobuten, ein Polystyrol, ein Ethylenpropylene, ein Mischpolymer davon oder eine Mischung davon ist.

10. Zubereitung nach einem der Ansprüche 1 bis 9, **dadurch gekennzeichnet, dass** sie zusätzlich weitere kosmetische oder dermatologische Wirkstoffe enthält.

11. Zubereitung nach Anspruch 10, **dadurch gekennzeichnet, dass** sie zusätzlich Pigmente oder UV-Filter enthält.

12. Zubereitung nach einem der Ansprüche 1 bis 11, **dadurch gekennzeichnet, dass** sie 16 Gew.% Isodecan, 65 Gew.% Isoeicosan, 12 Gew.% Polyethylen, 5 Gew.% Harnstoff und 2 Gew.% Phytantriol enthält.

13. Verwendung von Phytantriol zur Verbesserung der hautsensorischen Eigenschaften von wasserfreien, harnstoffhaltigen, dermatologischen oder kosmetischen Zubereitungen.

## Claims

1. Anhydrous dermatological or cosmetic preparation containing urea, **characterized in that** it further comprises phytantriol.

2. Preparation according to claim 1, **characterized in that** it comprises a gel-like basis, which is essentially free of straight-chained hydrocarbons comprising 5 to 50 carbon atoms.

3. Preparation according to claim 2, **characterized in that** the gel-like basis comprises at least one liquid branched hydrocarbon and at least one solid polymer hydrocarbon.

4. Preparation according to any one of claims I to 3, **characterized in that** the urea is micronized,

5. Preparation according to any one of claims I to 4, **characterized in that** the concentration of phytantriol in the preparation is between 0,1 and 10 weight per cent.

6. Preparation according to any one of claims 1 to 4, **characterized in that** the concentration of phytantriol in the preparation is between 0,2 to 3 weight per cent.

7. Preparation according to any one of claims 1 to 6, **characterized in that** the concentration of urea in the preparation is between 3 and 50 weight per cent.

8. Preparation according to any one of claims 1 to 6, **characterized in that** the concentration of urea in the preparation is between 3 and 20 weight per cent.

9. Preparation according to any one of claims 1 to 8, **characterized in that** the branched aliphatic hydrocarbon is isoparaffine C11-C13, paraffin oil, a hydrated polydecene, an isododecane, an isohexadecane, an isocicosane or a compound thereof, and that the solid polymer hydrocarbon is a polyethylene, a polypropylene, a polybutene, a polyisobutene, a polystyrene, an ethylenepropylene, a copolymer thereof or a mixture thereof.

10. Preparation according to any one of claims 1 to 9, **characterized in that** it further comprises further cosmetic or dermatological agents.

11. Preparation according to claim 10, **characterised in that** it further comprises pigments or UV-filters.

12. Preparation according to any one of claims 1 to 11, **characterized in that** it comprises 16 weight per cent of isodecane, 65 weight per cent of isoeicosane, 12 wight per cent of polyethylene, 5 weight per cent of urea and 2 weight per cent of phytantriol.

13. Use of phytantriol for the improvement of the dermal sensory attributes of anhydrous, urea containing, dermatological or cosmetic preparations.

## Revendications

1. Préparation dermatologique ou cosmétique exempte d'eau et contenant de l'urée, **caractérisée en ce qu'**elle contient du phytantriol.

2. Préparation selon la revendication 1, **caractérisée en ce qu'**elle présente une base de type gel essentiellement exempte d'hydrocarbures non ramifiés comptant de 5 à 50 atomes de carbone.

3. Préparation selon la revendication 2, **caractérisée en ce que** la base de type gel contient au moins un hydrocarbure liquide à chaîne ramifiée et au moins un hydrocarbure polymère solide.

4. Préparation selon l'une des revendications 1 à 3, **caractérisée en ce que** l'urée est micronisée.

5. Préparation selon l'une des revendications 1 à 4, **caractérisée en que** le phytantriol présente dans la préparation une concentration comprise entre 0,1 et 10 % en poids.

6. Préparation selon l'une des revendications 1 à 4, **caractérisée en ce que** le phytantriol présente dans la préparation une concentration de 0,2 à 3 % en poids.

7. Préparation selon l'une des revendications 1 à 6, **caractérisée en ce que** l'urée présente dans la préparation une concentration de 3 à 50 % en poids.

8. Préparation selon l'une des revendications 1 à 6, **caractérisée en ce que** l'urée présente dans la préparation une concentration de 3 à 20 % en poids.

9. Préparation selon l'une des revendications 1 à 8, **caractérisée en ce que** l'hydrocarbure aliphatique ramifié est une isoparaffine en C₁₁ à C₁₃, une huile paraffinique, un polydécène hydrogéné, un isododécane, un isohexadécane, un isoéicosan ou un de leurs mélanges et **en ce que** l'hydrocarbure polymère solide est un polyéthylène, un polypropylène, un polybutène, un polyisobutène, un polystyrène, un copolymère d'éthylène et de propylène, un polymère mixte de ces monomères ou un mélange de ces polymères.

10. Préparation selon l'une des revendications 1 à 9, **caractérisée en ce qu'**elle contient en outre d'autres substances à action cosmétique ou dermatologique.

11. Préparation selon la revendication 10, **caractérisée en ce qu'**elle contient en outre des pigments ou des filtres UV.

12. Préparation selon l'une des revendications 1 à 11, **caractérisée en ce qu'**elle contient 16 % en poids d'isodécane, 65 % en poids d'isoéicosan, 12 % en poids de polyéthylène, 5 % en poids d'urée et 2 % en poids de phytantriol.

13. Utilisation de Phytantriol pour améliorer les propriétés de sensibilisé de la peau à des préparations dermatologiques ou cosmétiques exemptes d'eau et contenant de l'urée.
